# EUROPEAN PATENT APPLICATION

(11) **EP 3 992 281 A1**
(43) Date of publication of application: **04.05.2022**
(21) Application number: 20831615.8
(22) Date of filing: 29.06.2020
(51) Int. Cl.: C12N 7/00, C12N 9/12, A61K 31/522, A61K 35/768, A61K 38/45, A61P 35/00

(54) **ONCOLYTIC VIRUS WITH IMPROVED SAFETY AND ANTICANCER EFFECTS**

(30) Priority: 27.06.2019 KR 20190077377
(71) Applicant: Bionoxx Inc., Seongnam-si, Gyeonggi-do 13554 (KR)
(72) Inventor: HWANG, Taeho, Yangsan-si, Gyeongsangnam-do 50613 (KR); CHO, Mong, Yangsan-si, Gyeongsangnam-do 50613 (KR)
(74) Representative: Dehns
(86) International application number: PCT/KR2020/008472
(87) International publication number: WO 2020/263059

(57) **Abstract**

The present invention relates to an oncolytic virus with improved safety and anticancer effects, and a use thereof. The oncolytic virus with improved safety and anticancer effects according to the present invention can express an HSV-TK fragment which contains an effector domain composed of a minimum amino acid sequence capable of phosphorylating GCV or ACV while having no thymidine kinase (TK) activity, or a variant thereof to phosphorylate GCV or ACV, thereby killing cancer cells infected with the oncolytic virus and even neighboring cancer cells. In addition, GCV or ACV is involved in the suppression of viral proliferation and thus, can regulate virus-induced side effects even upon the administration of a high dose of the virus. Furthermore, an anticancer effect is increased even though the number of viral particles is reduced due to suppression of GCV against viral proliferation. Therefore, the oncolytic virus with improved safety and anticancer effects according to the present invention can be advantageously used for treating cancer.

## Description

### Technical Field

The present invention relates to an oncolytic virus with improved safety and anticancer effect, and to a use thereof.

### Background Art

With full-scale use of gene recombination techniques, clinical studies using oncolytic viruses with increased tumor selectivity and anticancer efficacy have been initiated. The first recombinant oncolytic virus reported in literature was a herpes simplex virus. Since then, studies on oncolysis using other viruses have been actively conducted.

The usefulness of oncolytic viruses has recently been drawing increasing attention as herpes virus-based T-Vec (Talimogene laherparepvec) was successfully commercialized for therapy of advanced melanoma in the United States and Europe. On the other hand, a thymidine kinase (TK) gene-deficient vaccinia virus has great clinical usefulness; however, the virus has a limit in maximizing its clinical effect due to a narrow therapeutic window. For the TK-deficient vaccinia virus, the narrow therapeutic window means that a high viral dose has great clinical efficacy but may entail clinical risks due to toxicity of the virus.

In fact, the phase II clinical trial of Pexa-Vec (JX-594; SillaJen, Inc.), which was conducted on 30 patients with primary liver cancer, showed clinical results that a high-dose group (10⁹ pfu) had an increased survival rate as compared with a low-dose group (10⁸ pfu). However, dose limiting toxicity (DLT) was observed at 3×10⁹ pfu in the phase I clinical trial, which was conducted with intratumoral administration, and this caused the maximum tolerable dose (MTD) to be limited to 1×10⁹ pfu. It has been reported that there was no relevance to the drug. However, early death after treatment with oncolytic viruses has been frequently reported, indicating that undesirable virus proliferation may lead to unpredictable results. These dose-dependent increase in efficacy and dose-limiting toxicity imply that there is a need to develop a safer and more effective vaccinia virus.

Meanwhile, ganciclovir (GCV) is an antiviral agent effective against herpes simplex virus, cytomegalovirus, and varicella zoster virus. In a case where GCV binds to TK of herpes simplex virus, 5'-end thereof is phosphorylated and converted into triphosphate-ganciclovir (GCV-TP). GCV-TP inhibits activity of DNA polymerase and attaches to the 3'-end of viral DNA so that DNA elongation is terminated. GCV-TP, which is a highly toxic substance, can block DNA synthesis even in cells, thereby exhibiting cytotoxicity.

Recently, the anticancer research has been conducted in which HSV1-TK is inserted into an oncolytic virus and the resulting oncolytic virus is co-administered with GCV to induce tumor cell death. According to the research, primarily, the oncolytic virus infects tumor cells to induce a direct anticancer effect; and GCV phosphorylated by HSV1-TK (suicide gene) inhibits tumor cell proliferation, thereby exhibiting an additional anticancer effect (Oliver W et al., Human Gene Therapy, Vol.10, No.16, 1999). The HSV1-TK/GCV system was mainly used for oncolytic virus therapies in which adenovirus is used as a vector. However, the additional cytotoxic effect anticipated by co-administration of GCV is still controversial.

Specifically, it was observed that in a case where an oncolytic virus obtained by inserting HSV-TK gene into a replication-competent adenovirus was co-administered with GCV, a cytotoxic effect was significantly increased in glioma cells. On the other hand, in many other studies in which HSV-TK was inserted into a replication-competent adenovirus, an additional anticancer effect caused by administration of GCV has not been consistently shown (Lambright ES et al., Gene Ther, 8: 946-53). This is reported to be because the HSV-TK/GCV system is involved not only in inhibition of tumor cell proliferation but also in inhibition of virus proliferation so that these effects are opposite and offset each other.

Therefore, there is a need to conduct studies for particular methods capable of enhancing an anticancer effect while ensuring safety, in applying the HSV-TK/GCV system to oncolytic viruses.

### Disclosure of Invention

### Technical Problem

Accordingly, as a result of conducting studies to develop an oncolytic virus with improved safety and anticancer effect, the present inventors have developed an oncolytic virus that has no thymidine kinase (TK) activity and can phosphorylate GCV or ACV, and have identified that this oncolytic virus exhibits excellent safety and anticancer effect in a case of being co-administered with GCV, thereby completing the present invention.

### Solution to Problem

To achieve the above-mentioned object, in an aspect of the present invention, there is provided an oncolytic virus, comprising a nucleotide sequence that encodes a polypeptide including an effector domain, wherein the effector domain is represented by SEQ ID NO: 1 and derived from herpes simplex virus thymidine kinase (HSV-TK).

In another aspect of the present invention, there is provided a pharmaceutical composition for preventing or treating cancer, comprising the oncolytic virus as an active ingredient.

In yet another aspect of the present invention, there is provided a pharmaceutical composition for preventing or treating cancer, comprising, as active ingredients, the oncolytic virus and ganciclovir (GCV) or aciclovir (ACV).

In still yet another aspect of the present invention, there is provided a method for producing an oncolytic vaccinia virus that includes a gene encoding mutated HSV-TK, the method comprising steps of: i) removing TK gene from a vaccinia virus and allowing the vaccinia virus to recombine with wild-type HSV-TK gene; and ii) performing continuous subculture of the recombinant vaccinia virus in the presence of bromodeoxyuridine (BrdU) in a host cell.

### Advantageous Effects of Invention

The oncolytic virus with improved safety and anticancer effect according to the present invention can express an HSV-TK fragment, which includes an effector domain composed of a minimum amino acid sequence capable of phosphorylating GCV or ACV while having no thymidine kinase (TK) activity, or a variant thereof to phosphorylate GCV or ACV, thereby killing cancer cells infected with the oncolytic virus and even neighboring cancer cells. In addition, GCV or ACV is also involved in inhibition of virus proliferation, and thus can regulate virus-induced side effects even upon administration of a high dose of the virus. Furthermore, the oncolytic virus exhibits an increased anticancer effect even though the number of viral particles is reduced due to inhibition of virus proliferation caused by GCV Therefore, the oncolytic virus with improved safety and anticancer effect according to the present invention can be effectively used for treating cancer.

### Brief Description of Drawings

FIG. 1 illustrates a schematic diagram showing a process for producing a mutated vaccinia virus.
FIG. 2 illustrates results, identifying that mutated vaccinia viruses (C1 to C5), each of which is an embodiment of a mutated vaccinia virus, express an HSV1-TK fragment.
FIG. 3 illustrates results, identifying that mutated vaccinia viruses (C6 to C10), each of which is an embodiment of a mutated vaccinia virus, express an HSV1-TK fragment.
FIG. 4 illustrates results, identifying that mutated vaccinia viruses (C11 to C20), each of which is an embodiment of a mutated vaccinia virus, express an HSV1-TK fragment.
FIG. 5 illustrates results, identifying that mutated vaccinia viruses (C21 to C30), each of which is an embodiment of a mutated vaccinia virus, express an HSV1-TK fragment.
FIG. 6 illustrates results, identifying that mutated vaccinia viruses (C31 to C40), each of which is an embodiment of a mutated vaccinia virus, express an HSV1-TK fragment.
FIG. 7 illustrates results, identifying that mutated vaccinia viruses (C41 to C50), each of which is an embodiment of a mutated vaccinia virus, express an HSV1-TK fragment.
FIG. 8 illustrates results, identifying that mutated vaccinia viruses (C51 to C55), each of which is an embodiment of a mutated vaccinia virus, express an HSV1-TK fragment.
FIG. 9 illustrates results, identifying that mutated vaccinia viruses (C56 to C60), each of which is an embodiment of a mutated vaccinia virus, express an HSV1-TK fragment.
FIG. 10 illustrates results, identifying that mutated vaccinia viruses (C61 to C70), each of which is an embodiment of a mutated vaccinia virus, express an HSV1-TK fragment.
FIG. 11 illustrates results, identifying that mutated vaccinia viruses (C71 to C80), each of which is an embodiment of a mutated vaccinia virus, express an HSV1-TK fragment.
FIG. 12 illustrates results, identifying that mutated vaccinia viruses (C81 to C90), each of which is an embodiment of a mutated vaccinia virus, express an HSV1-TK fragment.
FIG. 13 illustrates results, identifying that mutated vaccinia viruses (C91 to C100), each of which is an embodiment of a mutated vaccinia virus, express an HSV1-TK fragment.
FIG. 14 illustrates a schematic diagram showing the HSV1-TK fragments expressed by mutated vaccinia viruses (C1, C3, C52, C40/45, C57, WOTS-418, and C19), each of which is an embodiment of a mutated vaccinia virus.
FIG. 15 illustrates a graph, identifying luciferase activity in a supernatant separated from a culture solution of HeLa cancer cell line that has been treated with a shuttle plasmid vector and a Western reserve strain vaccinia virus.
FIG. 16 illustrates a graph, identifying luciferase activity in a pellet separated from a culture solution of HeLa cancer cell line that has been treated with a shuttle plasmid vector and a Western Reserve strain vaccinia virus.
FIG. 17 illustrates a graph, identifying luciferase activity in a supernatant separated from a culture solution of HeLa cancer cell line that has been treated with a shuttle plasmid vector and a Wyeth strain vaccinia virus.
FIG. 18 illustrates results, identifying that administration of a mutated vaccinia virus (C1, C3, C19, C45, or C52), which is an embodiment of a mutated vaccinia virus, and GCV to NCI-H460 cancer cell line causes decreased virus proliferation.
FIG. 19 illustrates results, identifying that administration of a mutated vaccinia virus (C40 or C57), which is an embodiment of a mutated vaccinia virus, and GCV to HeLa cancer cell line causes decreased virus proliferation.
FIG. 20 illustrates results, identifying that administration of a mutated vaccinia virus (C40 or C57), which is an embodiment of a mutated vaccinia virus, and GCV to NCI-H460 cancer cell line causes decreased virus proliferation.
FIG. 21 illustrates results, identifying that administration of a mutated vaccinia virus (C1, C3, C19, C45, or C52), which is an embodiment of a mutated vaccinia virus, and GCV to NCI-H460 cancer cell line causes increased cytotoxicity.
FIG. 22 illustrates results, identifying that co-administration of a mutated vaccinia virus (C40 or C57), which is an embodiment of a mutated vaccinia virus, and GCV to HeLa cancer cell line causes increased cytotoxicity.
FIG. 23 illustrates results, identifying that co-administration of a mutated vaccinia virus (C40 or C57), which is an embodiment of a mutated vaccinia virus, and GCV to NCI-H460 cancer cell line causes increased cytotoxicity.
FIG. 24 illustrates a graph obtained by treating 10 cancer cell lines with a mutated vaccinia virus (WOTS-418), and then observing cell viability of each cancer cell line.
FIG. 25 illustrates results, identifying that co-administration of a mutated vaccinia virus (WOTS-418), which is an embodiment of a mutated vaccinia virus, and ACV, GCV, or BrdU to A549 cancer cell line causes decreased virus proliferation.
FIG. 26 illustrates results, identifying that co-administration of a mutated vaccinia virus (WOTS-418), which is an embodiment of a mutated vaccinia virus, and GCV to NCI-H460 cancer cell line causes decreased virus proliferation.
FIG. 27 illustrates a graph obtained by performing co-administration of a mutated vaccinia virus (WOTS-418) and GCV to an HCT-116 cancer cell line-transplanted mouse model, sacrificing the mice, and then counting the number of virus particles detected in tumor tissues of the mice.
FIG. 28 illustrates results, identifying that co-administration of a mutated vaccinia virus (WOTS-418), which is an embodiment of a mutated vaccinia virus, and GCV to NCI-H460 cancer cell line causes increased cytotoxicity.
FIG. 29 illustrates results, identifying that co-administration of a mutated vaccinia virus (OTS-418), which is an embodiment of a mutated vaccinia virus, and GCV to NCI-H460 cancer cell line causes increased cytotoxicity.
FIG. 30 illustrates a schematic diagram showing an experimental schedule for identifying, with a human breast cancer cell line (MDA-MD-231)-transplanted mouse model, an anticancer effect caused by co-administration of a mutated vaccinia virus and hydroxyurea.
FIG. 31 illustrates results obtained by performing individual administration or co-administration of a mutated vaccinia virus (WOTS-418) and hydroxyurea to human breast cancer cell line-transplanted mice, and then measuring tumor volumes in the mice.
FIG. 32 illustrates results obtained by performing individual administration or co-administration of a mutated vaccinia virus (WOTS-418) and hydroxyurea to mouse renal cancer cell line (Renca)-transplanted mice, and then measuring tumor volumes in the mice.
FIG. 33 illustrates a schematic diagram showing an experimental schedule for identifying, with a human colorectal cancer cell line (CT-26)-transplanted mouse model, an anticancer effect caused by co-administration of a mutated vaccinia virus and hydroxyurea.
FIG. 34 illustrates results obtained by performing individual administration or co-administration of a mutated vaccinia virus (WOTS-418) and hydroxyurea to human colorectal cancer cell line (CT-26)-transplanted mice, and then measuring tumor volumes in the mice.
FIG. 35 illustrates a graph obtained by performing individual administration or co-administration of a mutated vaccinia virus (WOTS-418) and hydroxyurea to human colorectal cancer cell line (CT-26)-transplanted mice, isolating CD8+ T cells from the mice, performing co-culture of the CD8+ T cells with cancer cells, and then measuring the number of CD8+ T cells that secrete INF-γ.
FIG. 36 illustrates photographs obtained by performing individual administration or co-administration of a mutated vaccinia virus (WOTS-418) and hydroxyurea to human colorectal cancer cell line (CT-26)-transplanted mice, isolating CD8+ T cells from the mice, performing co-culture of the CD8+ T cells with cancer cells, and then staining CD8+ T cells that secrete INF-γ.

### Best Mode for Carrying out the Invention

Hereinafter, the present invention will be described in detail.

In an aspect of the present invention, there is provided an oncolytic virus, comprising a nucleotide sequence that encodes a polypeptide including an effector domain, wherein the effector domain is represented by SEQ ID NO: 1 and derived from herpes simplex virus thymidine kinase (HSV-TK).

As used herein, the term "herpes simplex virus thymidine kinase (HSV-TK)" refers to an enzyme involved in an initial phosphorylation reaction during DNA synthesis in a herpes simplex virus. In addition, HSV-TK is also involved in phosphorylation of ganciclovir (GCV) or acyclovir (ACV), which is an antiviral agent. In particular, HSV1-TK responds to GCV or ACV about 10 times more sensitive than TK present in other viruses. The HSV may be herpes simplex virus type 1 (HSV1) or herpes simplex virus type 2 (HSV2). Specifically, the HSV may be HSV1. In addition, the HSV-TK may be herpes simplex virus type 1 thymidine kinase (HSV1-TK).

As used herein, the term "effector domain" refers to a protein domain consisting of consecutive amino acids at positions 1 to 145 in wild-type HSV-1TK that is represented by SEQ ID NO: 15 and consists of 376 amino acids. The effector domain has low or no TK activity as compared with the wild-type HSV-1TK, and thus an oncolytic virus, which includes a nucleotide sequence encoding a polypeptide including the effector domain, cannot proliferate itself. However, the effector domain includes an ATP binding site and is capable of phosphorylating GCV or ACV. In a case where a cell infected with an oncolytic virus, which includes a nucleotide sequence encoding a polypeptide including the effector domain, is treated with GCV or ACV, the GCV or ACV may be phosphorylated.

The polypeptide including the effector domain may further have 0 to 231 amino acids that are linked to the C-terminus of the effector domain. Here, the polypeptide has low or no TK activity as compared with the wild-type HSV-1TK, and thus an oncolytic virus that includes a nucleotide sequence encoding the polypeptide cannot proliferate itself. In addition, the polypeptide is capable of phosphorylating GCV or ACV In a case where a cell infected with an oncolytic virus, which includes the nucleotide sequence encoding the polypeptide, is treated with GCV or ACV, the GCV or ACV may be phosphorylated.

Specifically, the polypeptide including the effector domain may further have 0 to 231, 10 to 200, 20 to 150, or 40 to 100 amino acids which are linked to the C-terminus of the effector domain. Preferably, the polypeptide including the effector domain may further have 36, 82, or 231 amino acids that are linked to the C-terminus of the effector domain.

The polypeptide including the effector domain may consist of an amino acid sequence represented by SEQ ID NO: 1, 3, 5, 7, 9, 11, or 13. The nucleotide sequence encoding the polypeptide may be a nucleotide sequence encoding the amino acid sequence represented by SEQ ID NO: 1, 3, 5, 7, 9, 11, or 13. Specifically, the nucleotide sequence encoding the polypeptide may be a nucleotide sequence represented by SEQ ID NO: 2, 4, 6, 8, 10, 12, or 14.

To develop an oncolytic virus with improved safety and anticancer effect, the present inventors produced a recombinant vaccinia virus including the wild-type HSV1-TK gene, and then induced the virus to undergo adaptive evolution in the absence of TK. The vaccinia virus having undergone adaptive evolution was subjected to experiments of luciferase activity, genome analysis, and sensitivity for GCV. Through the experiments, mutated vaccinia viruses (C1, C3, C40, C45, C52, and C57) were selected which express an HSV1-TK fragment or a variant thereof. In addition, mutated vaccinia viruses (WOTS-418, OTS-418) were produced which include a gene encoding an HSV-TK variant obtained by mutation of a part of the amino acid sequence represented by SEQ ID NO: 15. Then, the nucleotide sequences of the mutated vaccinia viruses (C1, C3, C40, C45, C52, C57, and WOTS-418) were analyzed. As a result, it was identified that up to the amino acid at position 145 (reference point), the amino acid sequence of the wild-type HSV-1TK represented by SEQ ID NO: 15 phosphorylates GCV or ACV while having no TK activity.

As used herein, the term "oncolytic virus" refers to a recombinant virus, the gene of which has been manipulated to replicate specifically in cancer cells so that the virus can destroy the cancer cells. The oncolytic virus may be derived from adenovirus, herpes simplex virus, measles virus, lentivirus, retrovirus, cytomegalovirus, baculovirus, reovirus, adeno-associated virus, myxoma virus, vesicular stomatitis virus, poliovirus, Newcastle disease virus, parvovirus, coxsackievirus, senecavirus, vaccinia virus, or poxvirus. Preferably, the oncolytic virus may be derived from a vaccinia virus.

The vaccinia virus may be, but is not limited to, a vaccinia virus strain, that is, Western Reserve (WR), New York vaccinia virus (NYVAC), Wyeth (The New York City Board of Health; NYCBOH), LC16m8, Lister, Copenhagen, Tian Tan, USSR, TashKent, Evans, International Health Division-J (IHD-J), or International Health Division-White (IHD-W).

In another aspect of the present invention, there is provided a pharmaceutical composition for preventing or treating cancer, comprising, as an active ingredient, an oncolytic virus that comprises a nucleotide sequence encoding a polypeptide including an effector domain, wherein the effector domain is represented by SEQ ID NO: 1 and derived from herpes simplex virus thymidine kinase (HSV-TK).

The oncolytic virus, which is included as an active ingredient in the pharmaceutical composition, is as described above.

A dosage of the oncolytic virus varies depending on the individual's condition and body weight, the severity of disease, the type of drug, the route and period of administration, and can be appropriately selected by a person skilled in the art. The dosage may be such that a patient receives 1×10³ to 1×10¹⁸ of virus particles, infectious virus units (TCID₅₀), or plaque forming units (pfu). Specifically, the dosage may be such that a patient receives 1×10³, 2×10³, 5×10³, 1×10⁴, 2×10⁴, 5×10⁴, 1×10⁵, 2×10⁵, 5×10⁵, 1×10⁶, 2×10⁶, 5×10⁶, 1×10⁷, 2×10⁷, 5×10⁷, 1×10⁸, 2×10⁸, 5×10⁸ , 1×10⁹, 2×10⁹ , 5×10⁹ , 1×10¹⁰, 5×10¹⁰ , 1×10¹¹, 5×10¹¹, 1×10¹², 1×10¹³, 1×10¹⁴, 1×10¹⁵, 1 ×10¹⁶, 1×10¹⁷, 1×10¹⁸, or higher of virus particles, infectious virus units, or plaque forming units are administered, and various numerical values and ranges between the above-mentioned numerical values may also be included therein. Preferably, the oncolytic virus may be administered at a dose of 1×10³ to 1×10¹⁰ pfu.

The cancer may be any one selected from the group consisting of lung cancer, colorectal cancer, prostate cancer, thyroid cancer, breast cancer, brain cancer, head and neck cancer, esophageal cancer, skin cancer, thymic cancer, gastric cancer, colon cancer, liver cancer, ovarian cancer, uterine cancer, bladder cancer, rectal cancer, gallbladder cancer, biliary tract cancer, pancreatic cancer, non-small cell lung cancer, bone cancer, intraocular melanoma, perianal cancer, fallopian tube carcinoma, endometrial carcinoma, cervical cancer, vaginal carcinoma, vulvar carcinoma, Hodgkin's disease, small intestine cancer, endocrine adenocarcinoma, parathyroid cancer, adrenal cancer, soft tissue sarcoma, urethral cancer, penile cancer, chronic leukemia, acute leukemia, lymphocytic lymphoma, kidney cancer, ureteral cancer, renal cell carcinoma, renal pelvis carcinoma, central nervous system tumor, primary central nervous system lymphoma, spinal cord tumor, brainstem glioma, pituitary adenoma, and a combination thereof.

The pharmaceutical composition of the present invention may further comprise a physiologically acceptable carrier. In addition, the pharmaceutical composition of the present invention may further comprise suitable excipients and diluents commonly used in the preparation of pharmaceutical compositions. In addition, the pharmaceutical composition may be formulated in the form of an injection according to a conventional method.

In a case of being formulated as preparations for parenteral administration, the pharmaceutical composition may be formulated into sterilized aqueous solutions, non-aqueous solutions, suspensions, emulsions, freeze-dried preparations, suppositories, or the like. For the non-aqueous solution or the suspension, propylene glycol, polyethylene glycol, vegetable oil such as olive oil, injectable ester such as ethyl oleate, or the like may be used. As the base of the suppository, Witepsol^{™}, macrogol, Tween^{™} 61, cacao butter, laurin fat, glycerogelatin, or the like may be used.

Regarding the administration route, dosage, and frequency of administration, the pharmaceutical composition may be administered to a subject in a variety of ways and amounts depending on the patient's condition and the presence or absence of side effects; and the optimal administration route, dosage, and frequency of administration therefor may be selected by a person skilled in the art within a suitable range. In addition, the pharmaceutical composition may be administered in combination with another drug or physiologically active substance whose therapeutic effect is known for the disease to be treated, or may be formulated in the form of a combination preparation with the other drug.

The pharmaceutical composition may be administered parenterally, and such administration may be performed by any suitable method, such as intratumoral, intraperitoneal, subcutaneous, intradermal, intranodal, and intravenous administration. Among these, intratumoral, intraperitoneal, or intravenous administration may be preferred. On the other hand, the dosage of the pharmaceutical composition may be determined depending on the administration schedule, the total dosage, and the patient's health condition.

In yet another aspect of the present invention, there is provided a pharmaceutical composition for preventing or treating cancer, comprising, as active ingredients, an oncolytic virus and ganciclovir (GCV) or aciclovir (ACV), wherein the oncolytic virus comprising a nucleotide sequence that encodes a polypeptide including an effector domain, wherein the effector domain is represented by SEQ ID NO: 1 and derived from herpes simplex virus thymidine kinase (HSV-TK).

The oncolytic virus and the GCV or ACV, which are included in the pharmaceutical composition, may be administered simultaneously, or co-administered sequentially or in reverse order. Specifically, the oncolytic virus and the GCV or ACV, which are included in the pharmaceutical composition, may be administered simultaneously. In addition, administration of the pharmaceutical composition may be such that the oncolytic virus is administered first followed by the GCV or ACV, in which these ingredients are included in the pharmaceutical composition. In addition, administration of the pharmaceutical composition may be such that the oncolytic virus is administered first followed by the GCV or ACV, then the oncolytic virus again, in which these ingredients are included in the pharmaceutical composition.

The oncolytic virus, which is included as an active ingredient in the pharmaceutical composition, is as described above.

A dosage of the oncolytic virus varies depending on the individual's condition and body weight, the severity of disease, the type of drug, the route and period of administration, and can be appropriately selected by a person skilled in the art. The dosage may be such that a patient receives 1×10³ to 1×10¹⁸ of virus particles, infectious virus units (TCID₅₀), or plaque forming units (pfu). Specifically, the dosage may be such that a patient receives 1×10 , 2×10³ , 5×10³, 1×10⁴, 2×10⁴, 5×10⁴ , 1×10⁵, 2×10⁵, 5×10⁵, 1×10⁶, 2×10⁶, 5×10⁶, 1×10⁷, 2×10⁷, 5×10⁷, 1×10⁸, 2×10⁸, 5×10⁸ , 1×10⁹, 2×10⁹, 5×10⁹, 1×10¹⁰, 5×10¹⁰, 1×10¹¹, 5×10¹¹, 1×10¹², 1×10¹³, 1×10¹⁴, 1×10¹⁵, 1×10¹⁶, 1×10¹⁷, 1×10¹⁸, or higher of virus particles, infectious virus units (TCID50), or plaque forming units (pfu), and various numerical values and ranges between the above-mentioned numerical values may also be included therein. Preferably, the oncolytic virus may be administered at a dose of 1×10³ to 1×10¹⁰ pfu.

As used herein, the term "GCV" refers to an antiviral agent that is called ganciclovir and is effective against herpes simplex virus, cytomegalovirus, and varicella zoster virus. The GCV is phosphorylated at the 5'-end by TK of the virus and converted into ganciclovir triphosphate (GCV-TP). GCV-TP inhibits activity of the viral DNA polymerase and attaches to the 3'-end of the viral DNA so that DNA elongation can be terminated. In addition, the phosphorylated GCV can stop cellular DNA replication, and thus inhibit cell growth. The GCV is represented by Formula 1.

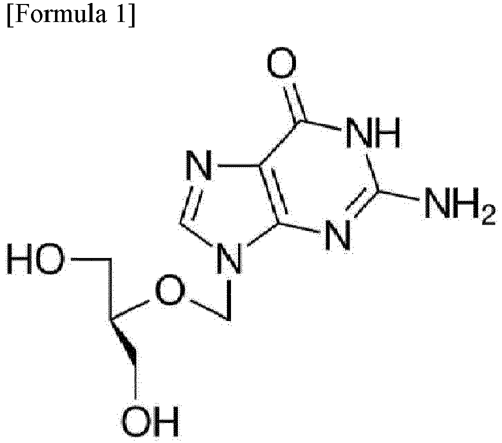

As used herein, the term "ACV" refers to an antiviral agent that is called acyclovir and is effective against herpes simplex virus, varicella zoster virus, and Epstein-Barr virus. The ACV is phosphorylated by TK of the virus and converted into aciclovir triphosphate (ACV-TP). ACV-TP inhibits activity of the viral DNA polymerase and attaches to the 3'-end of the viral DNA so that DNA elongation can be terminated. The ACV is represented by Formula 2.

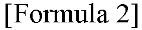

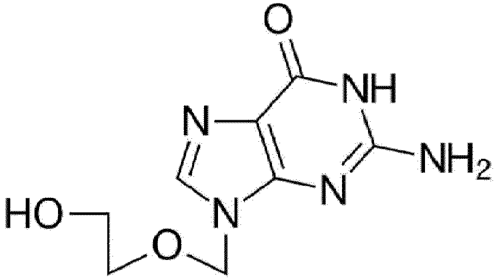

In addition, the GCV or ACV may be administered at a dose of 0.1 µg/kg to 50 mg/kg. Specifically, the GCV or ACV may be administered at a dose of 0.1 µg/kg to 50 mg/kg, 1 µg/kg to 40 mg/kg, 5 µg/kg to 30 mg/kg, or 10 µg/kg to 20 mg/kg.

The cancer may be any one selected from the group consisting of lung cancer, colorectal cancer, prostate cancer, thyroid cancer, breast cancer, brain cancer, head and neck cancer, esophageal cancer, skin cancer, thymic cancer, gastric cancer, colon cancer, liver cancer, ovarian cancer, uterine cancer, bladder cancer, rectal cancer, gallbladder cancer, biliary tract cancer, pancreatic cancer, non-small cell lung cancer, bone cancer, intraocular melanoma, perianal cancer, fallopian tube carcinoma, endometrial carcinoma, cervical cancer, vaginal carcinoma, vulvar carcinoma, Hodgkin's disease, small intestine cancer, endocrine adenocarcinoma, parathyroid cancer, adrenal cancer, soft tissue sarcoma, urethral cancer, penile cancer, chronic leukemia, acute leukemia, lymphocytic lymphoma, kidney cancer, ureteral cancer, renal cell carcinoma, renal pelvis carcinoma, central nervous system tumor, primary central nervous system lymphoma, spinal cord tumor, brainstem glioma, pituitary adenoma, and a combination thereof.

The pharmaceutical composition of the present invention may further comprise a physiologically acceptable carrier. In addition, the pharmaceutical composition of the present invention may further comprise suitable excipients and diluents commonly used in the preparation of pharmaceutical compositions. In addition, the pharmaceutical composition may be formulated in the form of an injection according to a conventional method.

In a case of being formulated as preparations for parenteral administration, the pharmaceutical composition may be formulated into sterilized aqueous solutions, non-aqueous solutions, suspensions, emulsions, freeze-dried preparations, suppositories, or the like. For the non-aqueous solution or the suspension, propylene glycol, polyethylene glycol, vegetable oil such as olive oil, injectable ester such as ethyl oleate, or the like may be used. As the base of the suppository, Witepsol^{™}, macrogol, Tween^{™} 61, cacao butter, laurin fat, glycerogelatin, or the like may be used.

Regarding the administration route, dosage, and frequency of administration, the pharmaceutical composition may be administered to a subject in a variety of ways and amounts depending on the patient's condition and the presence or absence of side effects; and the optimal administration route, dosage, and frequency of administration therefor may be selected by a person skilled in the art within a suitable range. In addition, the pharmaceutical composition may be administered in combination with another drug or physiologically active substance whose therapeutic effect is known for the disease to be treated, or may be formulated in the form of a combination preparation with the other drug.

The pharmaceutical composition may be administered parenterally, and such administration may be performed by any suitable method, such as intratumoral, intraperitoneal, subcutaneous, intradermal, intranodal, and intravenous administration. Among these, intratumoral, intraperitoneal, or intravenous administration may be preferred. On the other hand, the dosage of the pharmaceutical composition may be determined depending on the administration schedule, the total dosage, and the patient's health condition.

In still yet another aspect of the present invention, there is provided a method for treating cancer, comprising a step of administering an oncolytic virus and ganciclovir (GCV) or aciclovir (ACV), the oncolytic virus comprising a nucleotide sequence that encodes a polypeptide including an effector domain, wherein the effector domain is represented by SEQ ID NO: 1 and derived from herpes simplex virus thymidine kinase (HSV-TK).

The oncolytic virus, GCV, and ACV are as described above.

As used herein, the term "individual" refers to a person who has or is suffering from a disease in a state that can be alleviated, inhibited, or treated by administering the oncolytic virus of the present invention and GCV or ACV.

In still yet another aspect of the present invention, there is provided a use of an oncolytic virus for the treatment of cancer, the oncolytic virus comprising a nucleotide sequence encoding a polypeptide including an effector domain, wherein the effector domain is represented by SEQ ID NO: 1 and derived from herpes simplex virus thymidine kinase (HSV-TK).

In still yet another aspect of the present invention, there is provided a method for producing an oncolytic vaccinia virus that includes a gene encoding mutated HSV-TK, the method comprising steps of: i) removing TK gene from a vaccinia virus and allowing the vaccinia virus to recombine with wild-type HSV-TK gene; and ii) performing continuous subculture of the recombinant vaccinia virus in the presence of bromodeoxyuridine (BrdU) in a host cell.

The wild-type HSV-TK gene may be a nucleotide sequence represented by SEQ ID NO: 16.

The oncolytic vaccinia virus has low or no TK activity and can phosphorylate GCV or ACV.

The subculture may be performed continuously at least 3 times, and may preferably be performed continuously at least 10 times.

In still yet another aspect of the present invention, there is provided a pharmaceutical composition for preventing or treating cancer, comprising, as active ingredients, an oncolytic virus and hydroxyurea, the oncolytic virus comprising a nucleotide sequence that encodes a polypeptide including an effector domain, wherein the effector domain is represented by SEQ ID NO: 1 and derived from herpes simplex virus thymidine kinase (HSV-TK). For the oncolytic virus, see the above description.

As used herein, the term "hydroxyurea" refers to a compound having the following formula.

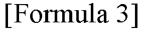

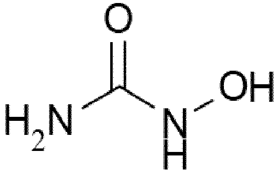

The hydroxyurea is known as an anticancer agent that inhibits DNA synthesis; however, the exact mechanism thereof is not elucidated. In addition, the hydroxyurea may be included in the pharmaceutical composition in the form of a commercialized drug that contains hydroxyurea. Examples of the commercialized drug that contains hydroxyurea may include, but are not limited to, Hydroxyurea^{®}, Hydrea^{®}, Droxia^{™}, Mylocel^{™}, Siklos^{®}, and Hydrine^{®} cap. The hydroxyurea can be taken orally, and parenteral administration thereof is also possible.

The oncolytic virus and the hydroxyurea, which are included in the pharmaceutical composition, may be administered simultaneously, or co-administered sequentially or in reverse order. Specifically, the oncolytic virus and the hydroxyurea may be administered simultaneously. In addition, the hydroxyurea may be administered first followed by the oncolytic virus. Furthermore, the oncolytic virus may be administered first followed by the hydroxyurea. In addition, the hydroxyurea may be administered first followed by the oncolytic virus, and then the hydroxyurea again.

In addition, the hydroxyurea may be administered at a dose of 1 mg/kg/day to 100 mg/kg/day, or 10 mg/kg/day to 90 mg/kg/day. Specifically, the hydroxyurea may be administered at a dose of 10 mg/kg/day to 90 mg/kg/day, 15 mg/kg/day to 80 mg/kg/day, 20 mg/kg/day to 70 mg/kg /day, 25 mg/kg/day to 65 mg/kg/day, or 30 mg/kg/day to 60 mg/kg/day. In an embodiment of the present invention, the hydroxyurea was administered at 30 mg/kg/day or 60 mg/kg/day. Depending on the dosage, the pharmaceutical composition may be administered in divided doses several times a day. Specifically, the pharmaceutical composition may be administered in divided doses, such as 1 to 4 times a day or 1 to 2 times a day.

The cancer may be any one selected from the group consisting of lung cancer, colorectal cancer, prostate cancer, thyroid cancer, breast cancer, brain cancer, head and neck cancer, esophageal cancer, skin cancer, thymic cancer, gastric cancer, colon cancer, liver cancer, ovarian cancer, uterine cancer, bladder cancer, rectal cancer, gallbladder cancer, biliary tract cancer, pancreatic cancer, non-small cell lung cancer, bone cancer, intraocular melanoma, perianal cancer, fallopian tube carcinoma, endometrial carcinoma, cervical cancer, vaginal carcinoma, vulvar carcinoma, Hodgkin's disease, small intestine cancer, endocrine adenocarcinoma, parathyroid cancer, adrenal cancer, soft tissue sarcoma, urethral cancer, penile cancer, chronic leukemia, acute leukemia, lymphocytic lymphoma, kidney cancer, ureteral cancer, renal cell carcinoma, renal pelvis carcinoma, central nervous system tumor, primary central nervous system lymphoma, spinal cord tumor, brainstem glioma, pituitary adenoma, and a combination thereof.

The pharmaceutical composition of the present invention may further comprise a physiologically acceptable carrier. In addition, the pharmaceutical composition of the present invention may further comprise suitable excipients and diluents commonly used in the preparation of pharmaceutical compositions. In addition, the pharmaceutical composition may be formulated in the form of an injection according to a conventional method.

In a case of being formulated as preparations for parenteral administration, the pharmaceutical composition may be formulated into sterilized aqueous solutions, non-aqueous solutions, suspensions, emulsions, freeze-dried preparations, suppositories, or the like. For the non-aqueous solution or the suspension, propylene glycol, polyethylene glycol, vegetable oil such as olive oil, injectable ester such as ethyl oleate, or the like may be used. As the base of the suppository, Witepsol^{™}, macrogol, Tween^{™} 61, cacao butter, laurin fat, glycerogelatin, or the like may be used.

Regarding the administration route, dosage, and frequency of administration, the pharmaceutical composition may be administered to a subject in a variety of ways and amounts depending on the patient's condition and the presence or absence of side effects; and the optimal administration route, dosage, and frequency of administration therefor may be selected by a person skilled in the art within a suitable range. In addition, the pharmaceutical composition may be administered in combination with another drug or physiologically active substance whose therapeutic effect is known for the disease to be treated, or may be formulated in the form of a combination preparation with the other drug.

The pharmaceutical composition may be administered parenterally, and such administration may be performed by any suitable method, such as intratumoral, intraperitoneal, subcutaneous, intradermal, intranodal, and intravenous administration. Among these, intratumoral, intraperitoneal, or intravenous administration may be preferred. On the other hand, the dosage of the pharmaceutical composition may be determined depending on the administration schedule, the total dosage, and the patient's health condition.

In still yet another aspect of the present invention, there is provided a method for treating cancer, comprising a step of administering the oncolytic virus.

In still yet another aspect of the present invention, there is provided a use of the oncolytic virus for the treatment of cancer.

In still yet another aspect of the present invention, there is provided a use of the oncolytic virus for the manufacture of a medicament for treating cancer.

In still yet another aspect of the present invention, there is provided a method for treating cancer, comprising a step of administering the pharmaceutical composition for preventing or treating cancer.

In still yet another aspect of the present invention, there is provided a use of the pharmaceutical composition for preventing or treating cancer for the treatment of cancer.

In still another aspect of the present invention, there is provided a use of the pharmaceutical composition for preventing or treating cancer for the manufacture of a medicament for treating cancer.

### Mode for the Invention

Hereinafter, the present invention will be described in more detail by way of the following examples. However, the following examples are for illustrative purposes only, and the scope of the present invention is not limited thereto.

### Example 1. Production of mutated vaccinia viruses

### Example 1.1. Construction of shuttle plasmid vector

The shuttle plasmid vector used was a shuttle plasmid vector produced by synthesizing a type 1 HSVTK gene (pSE/L promoter) and a firefly luciferase reporter (p7.5 promoter) gene and performing recombination of these genes into the pUC57amp+ plasmid (Genewiz, USA).

### Example 1.2. Construction of mutated vaccinia viruses (C1, C3, C40, C45, C52, and C57)

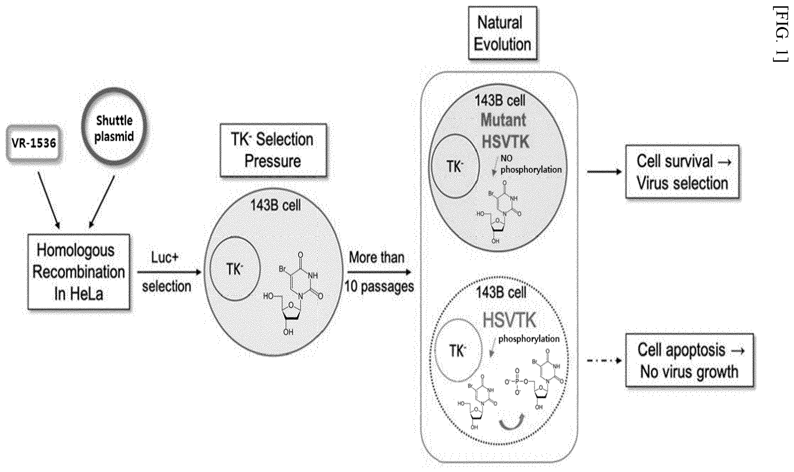

A process of producing mutated vaccinia viruses is illustrated in FIG. 1. Specifically, first, in order to obtain recombinant viruses, HeLa cells (ATCC) were seeded in 6-well plates at 4×10⁵ cells per well, and then EMEM medium containing 10% fetal bovine serum was added thereto. Treatment with Wyeth strain wild-type vaccinia virus (NYC Department of Health strain, WR-1536, ATCC) at an MOI of 0.05 was performed. After 2 hours, the medium was replaced with EMEM medium containing 2% fetal bovine serum, and Xfect^{™} polymer (Clonetech 631317, USA) was used to deliver into the cells 4 µg of the shuttle plasmid vector as constructed in Example 1.1. 4 hours after culture, the medium was replaced with EMEM medium containing 2% fetal bovine serum and the HeLa cells were further cultured for 72 hours. The recombinant vaccinia viruses containing HSV1-TK gene were obtained by checking luciferase activity in the HeLa cells.

Then, mutations that cause HSV1-TK lacking TK activity were induced by performing 10 consecutive subcultures in a state where a biochemical environment (TK- selection pressure) is applied which allows for selection of cells lacking TK function in the presence of BrdU (thymidine analogue, 15 µg/ml) in TK- osteosarcoma (143B TK-) cell line (ATCC). Lysates of mutated vaccinia virus clones having luciferase activity were dispensed on plates, and then 100 single plaques having luciferase activity were isolated. Subsequently, the single plaques were amplified, and then clones (S3C4#1_C1 to S3C4#1_C100) expressing HSV-TK fragments of different protein sizes were identified by Western blotting.

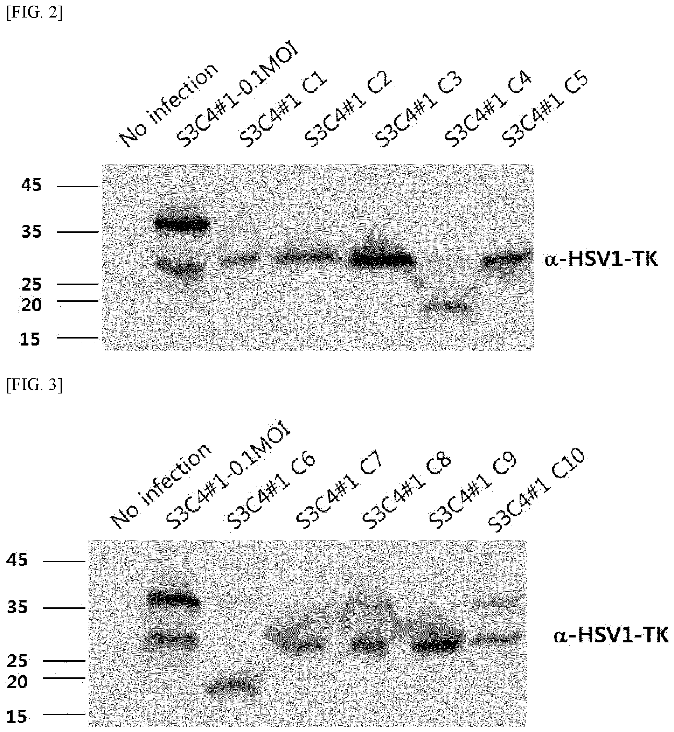

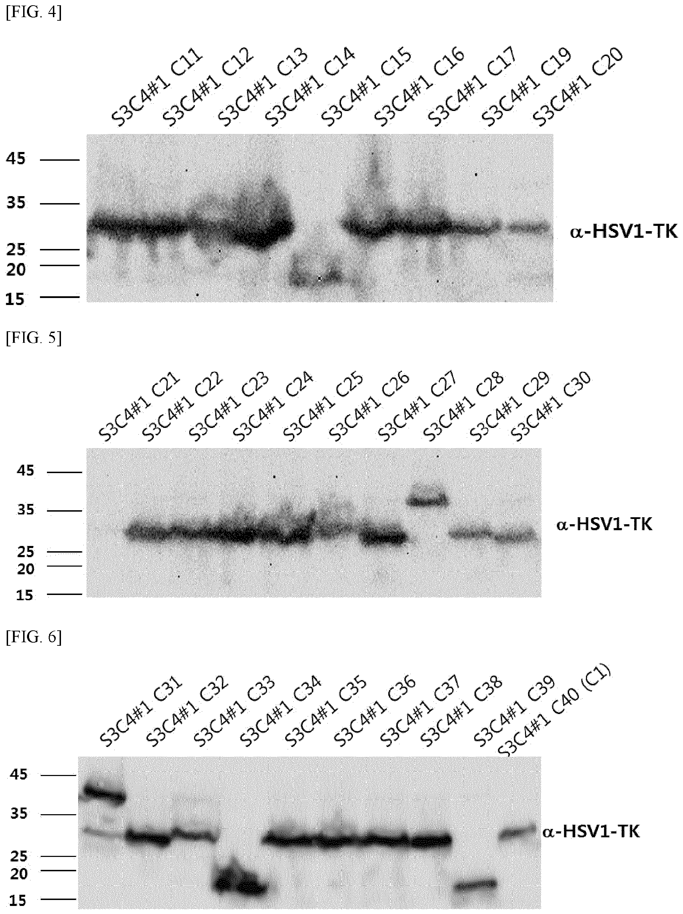

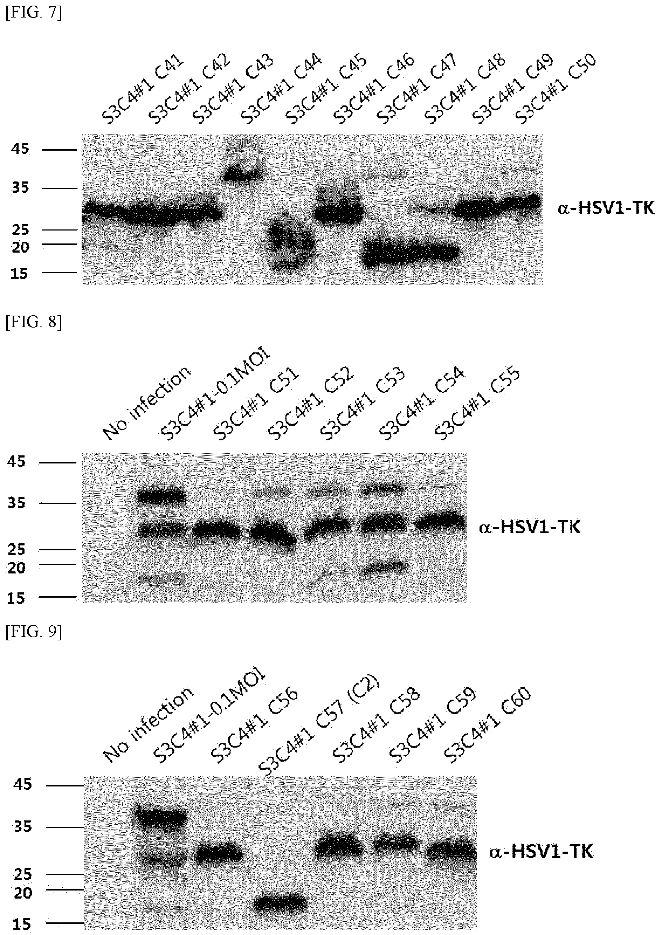

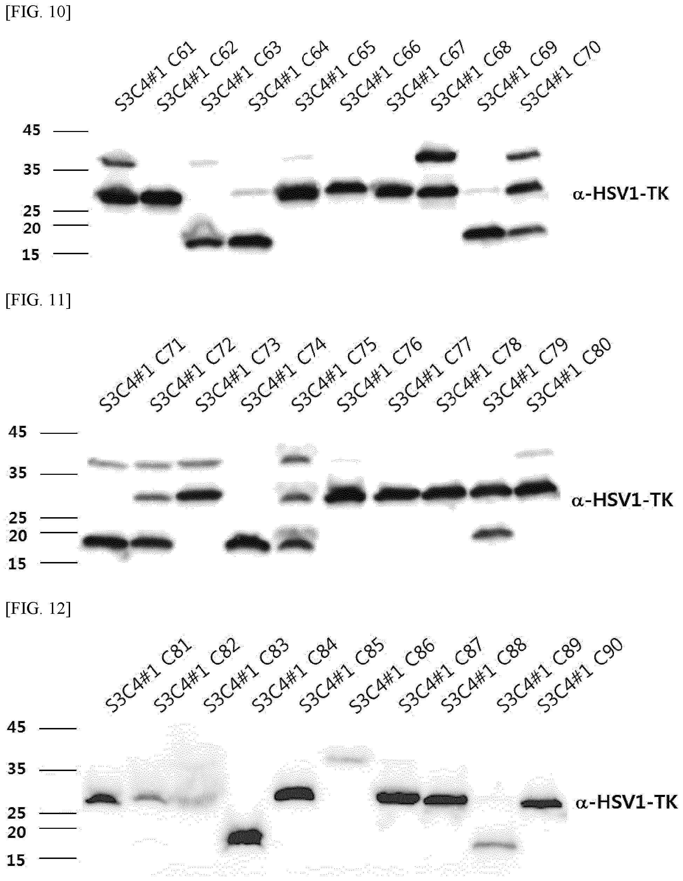

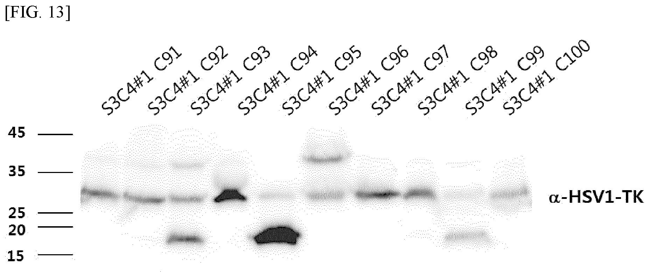

Specifically, treatment with the 100 clones at 15 µl each was performed to infect the HeLa cell line. Then, after 24 hours, the cells were collected and lysed to extract proteins. The extracted proteins were subjected to denaturation, and then electrophoresis was performed by loading 40 µg of each sample onto SDS-PAGE gel. After the electrophoresis, the sample was transferred to a PVDF membrane and reacted with anti-HSV1-TK antibody (Bethyl A50-101P) that is a primary antibody. Subsequently, the sample was washed with PBST, and then reacted with HRP-labeled anti-goat antibody (SantaCruz, sc-28037) that is a secondary antibody. The sample was washed again with PBST, treated with a chemiluminescent reagent, and checked using a Chemiluminescent Image system (Davinch K). As a result, it was identified that HSV1-TK fragment proteins were expressed in the viruses (FIGS. 2 to 13).

The 100 different clones basically do not have TK activity because they were cultured in a biochemical environment that allows for selection of cells lacking TK function. On the other hand, in order to screen clones with sensitivity to GCV, 10 clones, in which apoptosis occurred, were initially selected through real-time imaging while performing culture for 4 days in the presence of GCV in 96-well-plates, using Incucyte^{®} (Essen Biosciences) that is a real-time cell imaging and analysis system. In general, virus-induced cytotoxicity gradually increases after plaque formation. However, in a case of GCV-induced cytotoxicity, overall cytotoxicity occurs at once within 15 hours after plaque formation. Thus, the GCV-induced cytotoxicity was qualitatively checked.

In the colonies initially selected as a result of the image analysis, inhibition of virus proliferation and cytotoxicity caused by administration of GCV were checked. Then, the mutated vaccinia viruses in the colonies S3C4#1_C1, S3C4#1_C3, S3C4#1_C40, S3C4#1_45, S3C4#1_C52, and S3C4#1_C57 were finally selected. The mutated vaccinia viruses in the colonies S3C4#1_C1, S3C4#1_C3, S3C4#1_C40, S3C4#1_45, S3C4#1_C52, and S3C4#1_C57 were designated as "C1", "C3", "C40", "C45", "C52", and "C57", respectively.

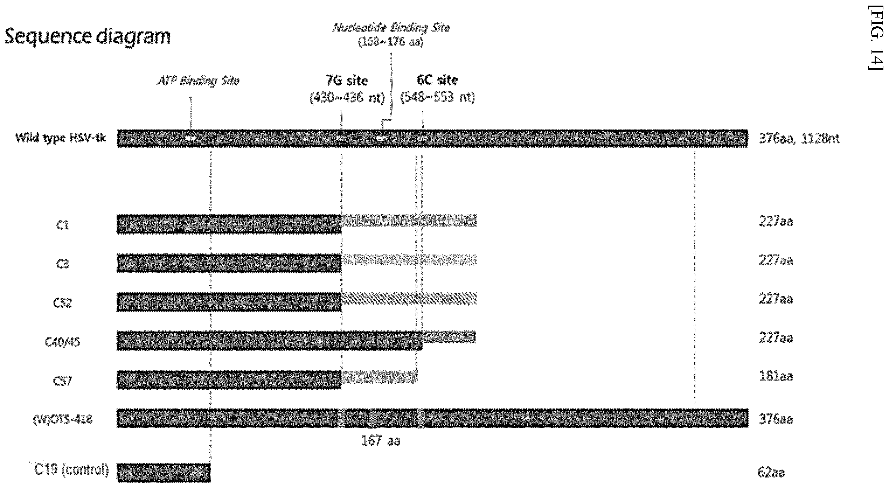

A request for amino acid sequencing of the C1, C3, C40, C45, C52, and C57 was made to Macrogen (Seoul, Korea). As a result, it was identified that the amino acid sequences of C40 and C45 are the same; and it was identified that C1, C3, C40/45, C52, and C57 have the amino acid sequences of SEQ ID NOs: 15, 17, 19, 21, 23, and 25. In particular, it was identified that in the HSV1-TKs of the selected mutated vaccinia viruses, a mutation occurred starting after the 145^{th} amino acid of the N-terminus (FIG. 14).

### Example 1.3. Production of mutated vaccinia viruses (WOTS-418/OTS-418)

The most reported mutations in the HSV-TKs are frameshift mutations caused by insertion or deletion of bases which occur in the nucleotide sequence sections at positions 430 to 436 (7 Gs) and at positions 548 to 583 (6 Cs). After the wild-type HSV-TK was inserted into the vaccinia viruses, 98% or higher of the mutations occurred in these sections. Accordingly, in order to cause a silent mutation in the nucleotide sequence sections, GGGGGGG, which is a nucleotide sequence at positions 430 to 436, was changed to GGTGGTG, and CCCCCC, which is a nucleotide sequence at positions 548 to 583, was changed to CCCCTC. In addition, the shuttle plasmid vector used was a shuttle plasmid vector produced by synthesizing a gene encoding an HSV-TK variant, which had been obtained from the amino acid sequence of HSV-TK of SEQ ID NO: 15 by substitution of alanine at position 167 with tyrosine, and a firefly luciferase reporter (p7.5 promoter) gene, and performing recombination of these genes into the pUC57amp+ plasmid (Genewiz, USA). Then, mutated vaccinia viruses were produced in the same manner as in Example 1.2 using the shuttle plasmid vector and the Western Reserve strain (ATCC) or Wyeth strain vaccinia virus. The mutated vaccinia virus produced using the Western Reserve strain vaccinia virus was designated as "WOTS-418", and the mutated vaccinia virus produced using the Wyeth strain vaccinia virus was designated as "OTS-418".

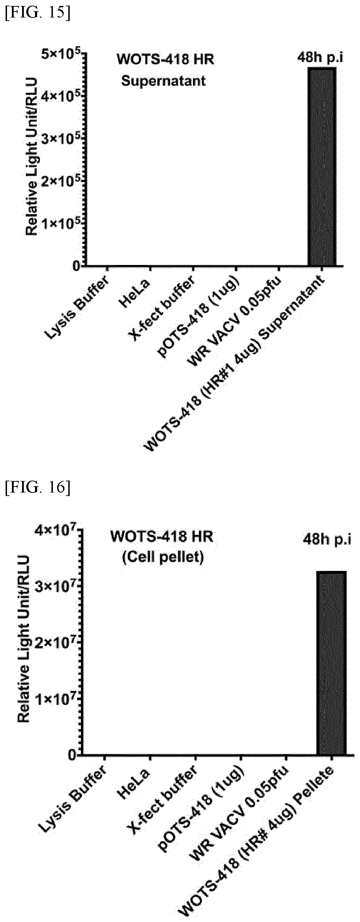

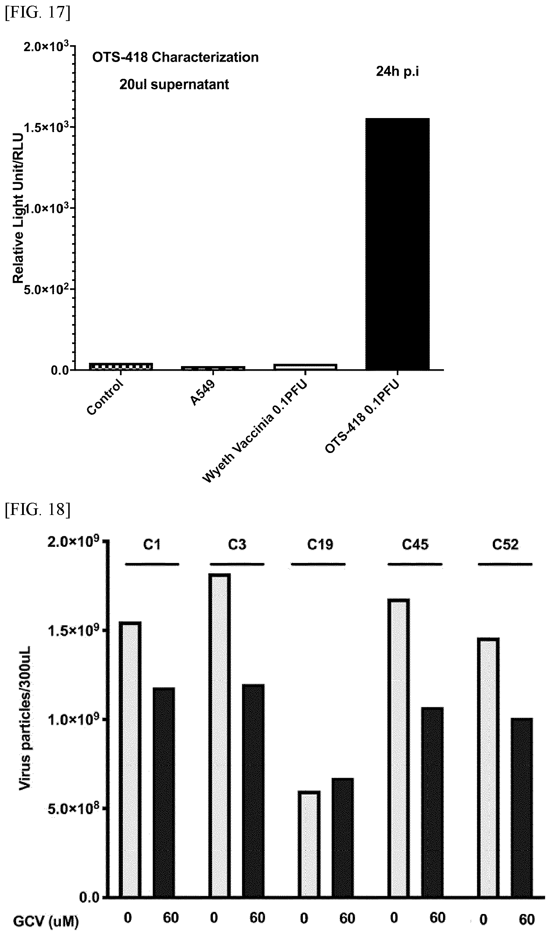

As a result, luciferase activity was identified in both the supernatant and the pellet which were separated from the culture solution of the HeLa cancer cell line treated with the shuttle plasmid vector and the Western Reserve strain vaccinia virus (FIGS. 15 and 16). In addition, luciferase activity was identified in the supernatant which was separated from the culture solution of the HeLa cancer cell line treated with the shuttle plasmid vector and the Wyeth strain vaccinia virus (FIG. 17). From these results, it was identified that the gene encoding the HSV-TK variant was introduced into the Western Reserve strain or Wyeth strain vaccinia virus.

### Example 2. Identification of inhibited proliferation of mutated vaccinia viruses (C1, C3, C45, C52) following administration of GCV (in vitro)

Inhibited proliferative capacity of C1, C3, C45, and C52 produced in Example 1.2, which is caused by administration of GCV, was checked. Here, the mutated vaccinia virus in the colony S3C4#1_C19 was used as a negative control, which was designated as "C19". In order to check a difference in the number of virus particles after subjecting the C1, C3, C19, C45, or C52 to treatment with GCV, quantitative polymerase chain reaction analysis (qPCR) was performed using *E9L* gene that is specifically expressed only by vaccinia virus.

Specifically, probes (SEQ ID NOs: 19 and 20) were prepared which recognize the *E9L* gene while binding to only one of the two complementary strands of DNA. The prepared probes were such that one luminescence is measured each time the virus proliferation occurs. The NCI-H460 cancer cell line was seeded at 1.5×10⁴ cells per well, and then infected with the virus C1, C3, C19, C45, or C52 at an MOI of 0.01 to 1. After 2 hours, co-treatment with GCV at a concentration of 60 µM was performed. Culture was performed for 48 hours, and then DNA was extracted using a virus extraction kit (QIAamp MinElute Virus Spin, QIAGEN, 57704). The extracted DNA was diluted to a concentration of 1 ng/5 µl and subjected to qPCR.

As a result, for C19, virus proliferation was not inhibited despite administration of GCV. On the other hand, it was identified that for C1, C3, C45, and C52, virus proliferation was inhibited following administration of GCV (FIG. 18). From these results, it was identified that C1, C3, C45, and C52 exhibited decreased proliferation capacity upon administration of GCV

### Example 3. Identification of decreased proliferation capacity of mutated vaccinia viruses (C40, C57) following administration of GCV (in vitro)

The proliferation capacity of C40 and C57 produced in Example 1.2 was checked upon administration of GCV In order to check a change in proliferation level of C40 or C57 in a case of being treated with GCV, quantitative polymerase chain reaction analysis (qPCR) was performed using the *E9L* gene.

Specifically, probes were prepared which recognize the *E9L* gene while binding to only one of the two complementary strands of DNA. The prepared probes were such that one luminescence is measured each time the virus proliferation occurs. The HeLa cancer cell line was seeded at 1×10⁴ cells per well or the NCI-H460 cancer cell line was seeded at 1.5×10⁴ cells per well. Then, the cells were infected with the virus C40 or C57 at an MOI of 0.1 (0.1 pfu/cells). After 2 hours, co-treatment with GCV at a concentration of 50 µM was performed. Culture was performed for 48 hours, and then DNA was extracted using the virus extraction kit. The extracted DNA was diluted to a concentration of 1 ng/5 µl and subjected to qPCR.

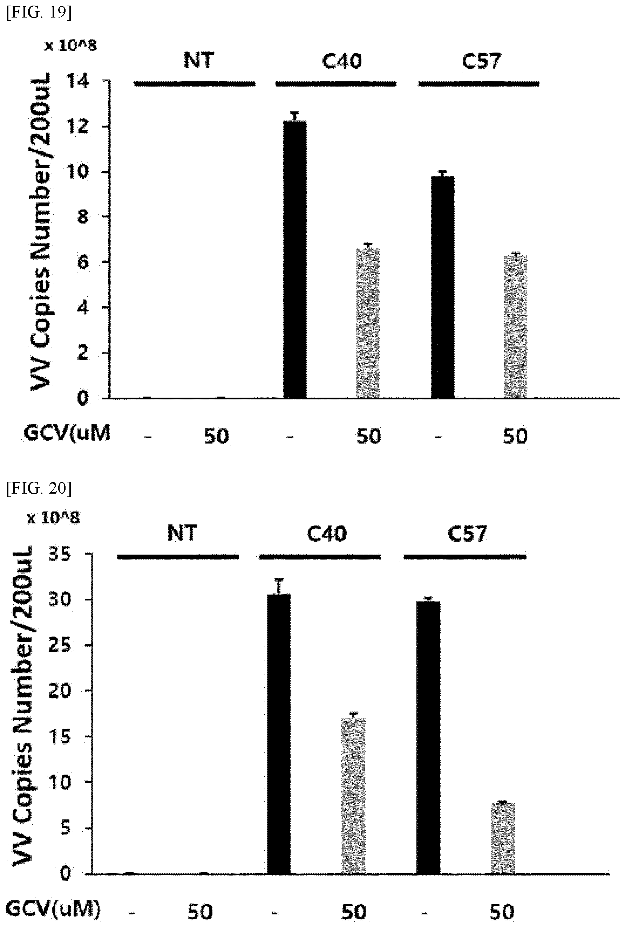

As a result, it was identified that for C40 and C57, virus proliferation was inhibited following administration of GCV (FIGS. 19 and 20). From these results, it was identified that C40 and C57 exhibited decreased proliferation capacity upon administration of GCV.

### Example 4. Identification of cytotoxicity of mutated vaccinia viruses (C1, C3, C45, C52) following administration of GCV (in vitro)

In order to identify whether although C1, C3, C45, and C52 exhibited inhibited proliferation due to GCV in Example 2, C1, C3, C45, and C52 maintain cytotoxicity upon administration of GCV, co-treatment of C1, C3, C19, C45, or C52 and GCV was performed for comparison of cytotoxicity. Specifically, the NCI-H460 cancer cell line was seeded at 1.5×10⁴ cells per well, and then infected with C1, C3, C19, C45, or C52 at an MOI of 0.01 to 1. After 2 hours, co-treatment with GCV at a concentration of 60 µM was performed. Culture was performed for 72 hours, and then cytotoxicity was analyzed using a CCK8 kit (Cell Counting Kit 8, Dojindo, Kumamoto, Japan).

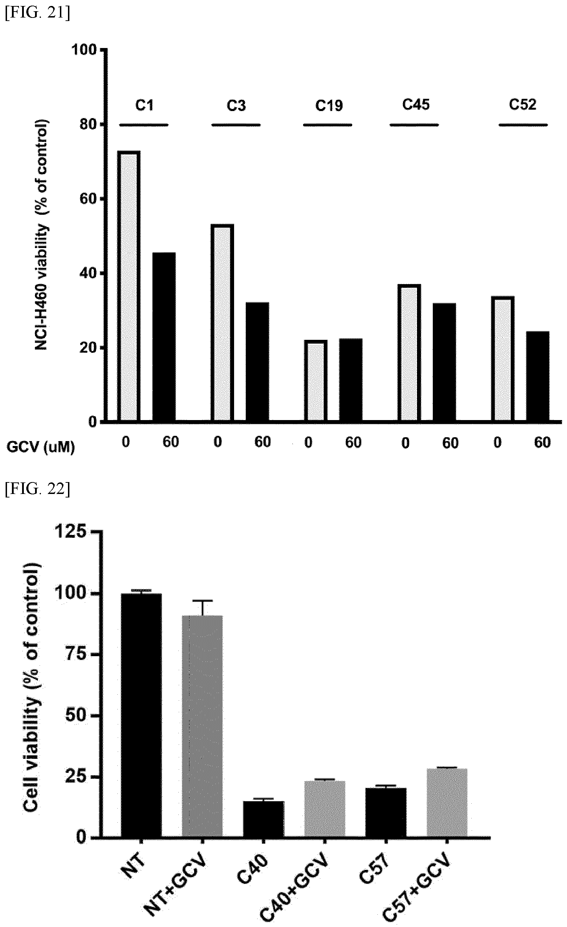

As a result, in a case where co-treatment of C1, C3, C45, or C52 and GCV was performed, the NCI-H460 cancer cell line was additionally killed by about 25% or higher, despite the GCV-induced virus proliferation inhibition in C1, C3, C45, and C52. On the other hand, in a case where co-treatment of C19 and GCV was performed, the NCI-H460 cancer cell line was killed to a degree similar to a case where treatment with only C19 was performed (FIG. 21). From these results, it was identified that cytotoxicity of C1, C3, C45, or C52 increased in a case where co-administration of C1, C3, C45, or C52 and GCV was performed.

### Example 5. Identification of cytotoxicity of mutated vaccinia viruses (C40, C57) following administration of GCV (in vitro)

In order to identify whether although C40 and C57 exhibited inhibited virus proliferation due to GCV in Example 3, C40 and C57 maintain cytotoxicity upon administration of GCV, co-treatment of C40 or C57 and GCV was performed to analyze their cytotoxicity.

Specifically, the HeLa cancer cell line was seeded at 1×10⁴ cells per well, or the NCI-H460 cancer cell line was seeded at 1.5×10⁴ cells per well. Then, co-treatment of C40 or C57 at an MOI of 0.1 (0.1 pfu/cells) and GCV at a concentration of 50 µM was performed on the cells for 2 hours for infection. Culture was performed for 48 hours, and then cytotoxicity was analyzed using a CCK8 kit (Cell Counting Kit 8).

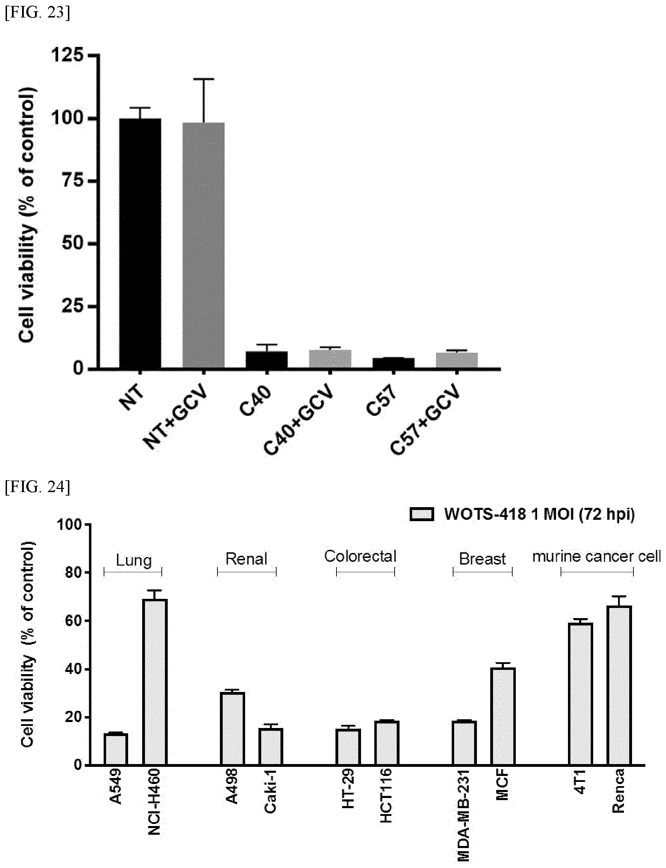

As a result, in a case where co-treatment of C40 or C57 and GCV was performed, the HeLa cancer cell line and NCI-H460 cancer cell line were killed to a similar extent to a case where treatment with only C40 or C57 was performed, despite the GCV-induced virus proliferation inhibition in C40 and C57 (FIGS. 22 and 23). From these results, it was identified that cytotoxicity of C40 or C57 increased in a case where co-administration of C40 or C57 and GCV was performed.

### Example 6. Identification of cytotoxicity of mutated vaccinia virus (WOTS-418) (in vitro)

In order to identify cytotoxicity of WOTS-418, which was produced in Example 1.3, against cancer cells, each of 10 cancer cell lines, that is, human lung cancer cell lines (A549, NCI-H460), human renal cancer cell lines (A498, Caki-1), human colorectal cancer cell lines (HT-29, HCT116), human breast cancer cell lines (MDA-MB-231, MCF), a mouse breast cancer cell line (4T1), and a mouse renal cancer cell line (Renca), was seeded in a 96-well-plate at 3×10³ cells per well, and then the cells were treated with WOTS-418 at an MOI of 1 (1 pfu/cells). Culture was performed for 72 hours, and then cytotoxicity was analyzed using a CCK8 kit (Cell Counting Kit 8). Here, the human lung cancer cell line (A549) and the mouse breast cancer cell line (4T1) were obtained from the American Type Culture Collection (ATCC). In addition, the human renal cancer cell line (A498), the human colorectal cancer cell lines (HT-29, HCT-116), the human lung cancer cell line (NCI-H460), the human breast cancer cell lines (MDA-MB-231, MCF), and the mouse renal cancer cell line (Renca) were obtained from the Korea Cell Line Bank (KCLB).

As a result, cytotoxicity of 60% or higher was observed for the human lung cancer cell line (A549), the human renal cancer cell lines (A498, Caki-1), the human colorectal cancer cell lines (HT-29, HCT-116), and human breast cancer cell lines (MDA-MB-231, MCF) (FIG. 24).

### Example 7. Identification of decreased proliferation capacity of mutated vaccinia virus (WOTS-418) following administration of ACV, GCV, or BrdU (in vitro)

The proliferative capacity of WOTS-418 produced in Example 1.3 was checked upon administration of GCV. In order to check a difference in the number of virus particles after subjecting WOTS-418 to treatment with GCV, quantitative polymerase chain reaction analysis (qPCR) was performed using the *E9L* gene.

Specifically, probes were prepared which recognize the *E9L* gene while binding to only one of the two complementary strands of DNA. The prepared probes were such that one luminescence is measured each time the virus proliferation occurs. The A549 cancer cell line was seeded at 1×10⁴ cells per well, and then infected with WOTS-418 at an MOI of 0.1 (0.1 pfu/cells). After 2 hours, co-treatment with ACV, GCV, or BrdU at a concentration of 200 µM or 300 µM was performed. Culture was performed for 48 hours, and then DNA was extracted using a virus extraction kit. The extracted DNA was diluted to a concentration of 1 ng/5 µl and subjected to qPCR.

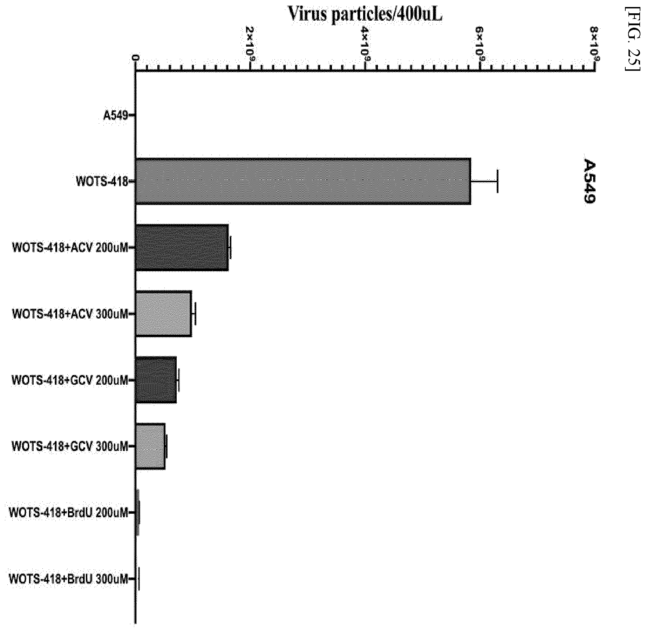

As a result, it was identified that for WOTS-418, virus proliferation was remarkably inhibited following co-administration of ACV, GCV, or BrdU (FIG. 25).

In addition, the NCI-H460 cancer cell line was seeded at 15×10⁴ cells per well, and then infected with WOTS-418 at an MOI of 0.1 (0.1 pfu/cells). After 2 hours, co-treatment with GCV at a concentration of 100 µM was performed. Culture was performed for 48 hours, and then DNA was extracted using the virus extraction kit. The extracted DNA was diluted to a concentration of 1 ng/5 µl and subjected to qPCR.

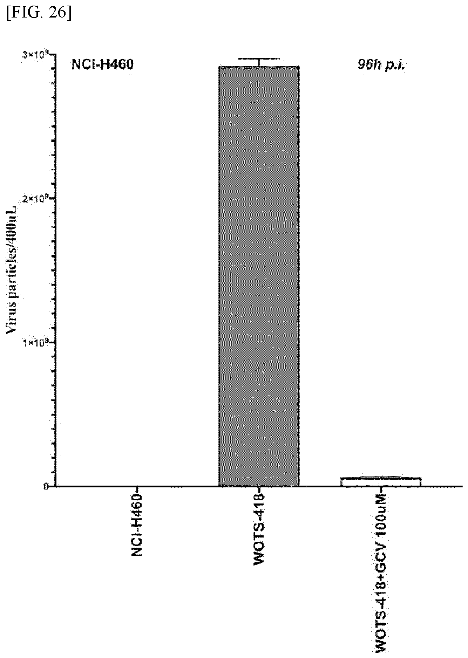

As a result, it was identified that for WOTS-418, virus proliferation was remarkably inhibited following co-administration of WOTS-418 and GCV (FIG. 26). From these results, it was identified that WOTS-418 had sensitivity to ACV, GCV, and BrdU, and exhibited decreased proliferation capacity upon administration of ACV, GCV, or BrdU.

### Example 8. Identification of decreased proliferation capacity of mutated vaccinia virus (WOTS-418) following administration of GCV (in vitro)

The proliferative capacity of WOTS-418 was checked by qPCR analysis in a human colorectal cancer cell line (HCT-116, 5×10⁶ cells/ml)-transplanted mouse (Balb/c nu/nu) model.

Specifically, mice (balb/c nu/nu) purchased from KOATECH (Korea) were subjected to a one-week acclimatization period, and then xenografted with the HCT-116 cancer cell line (Korea Cell Line Bank), which is a human colorectal cancer cell line, at 5×10⁶ cells. The tumor volume was observed until it reached 100 mm³ to 150 mm³, and then co-administration of WOTS-418 and GCV was performed intratumorally. Here, WOTS-418 (1×10⁶ pfu) was administered intraperitoneally. Starting after 4 days, GCV (50 mg/kg) was administered once a day for 3 days (D5, D6, D7). Starting from day 5, 3 mice per group were sacrificed and tumors were separated. The separated tumors were homogenized using a sample homogenizing system (OMNI Bead Ruptor 24), and then the number of virus particles was quantified through qPCR.

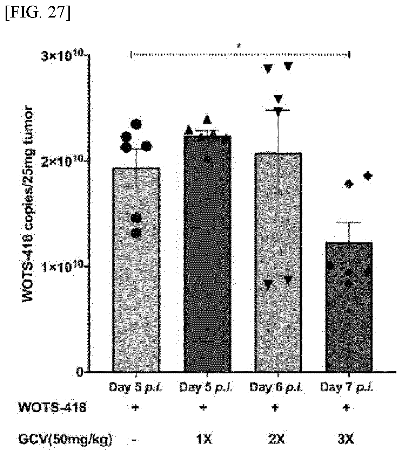

As a result, it was identified that virus proliferation inhibition caused by treatment with GCV occurred starting from the 3^{rd} day (FIG. 27).

### Example 9. Identification of cytotoxicity of mutated vaccinia virus (WOTS-418) (in vitro)

In order to identify whether although WOTS-418 exhibited inhibited proliferation due to GCV in Examples 7 and 8, this virus maintains cytotoxicity upon administration of GCV, co-treatment of WOTS-418 and GCV was performed for comparison of cytotoxicity. Specifically, the NCI-H460 cancer cell line was seeded at 1.5×10⁴ cells per well. Then, the cells were infected with WOTS-418 at an MOI of 0.02 (0.02 pfu/cells). After 2 hours, co-treatment with GCV at a concentration of 60 µM or 100 µM was performed. Culture was performed for 72 hours, and then cytotoxicity was analyzed using a CCK8 kit (Cell Counting Kit 8).

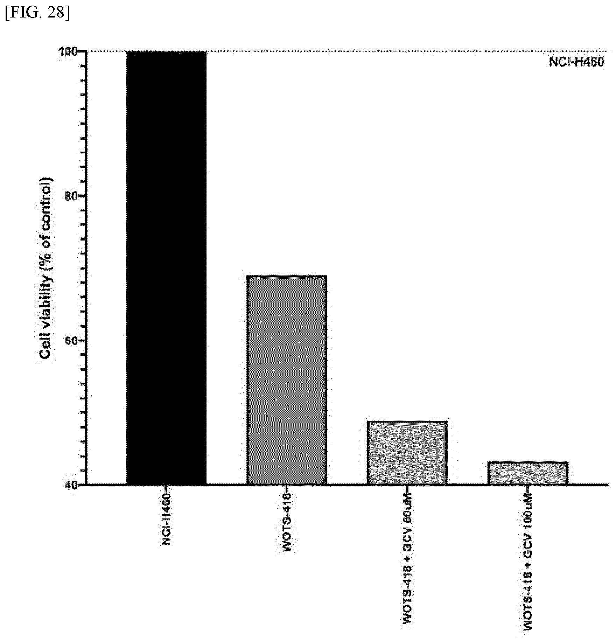

As a result, in a case where co-treatment of WOTS-418 and GCV was performed, the NCI-H460 cancer cell line was additionally killed by about 30% or higher, despite the GCV-induced virus proliferation inhibition in WOTS-418 (FIG. 28). From these results, it was identified that cytotoxicity of WOTS-418 increased in a case where co-administration of WOTS-418 and GCV was performed.

### Example 10. Identification of cytotoxicity of mutated vaccinia virus (OTS-418) (in vitro)

In order to identify cytotoxicity of OTS-418 upon administration of GCV, co-treatment of OTS-418 and GCV was performed for comparison of cytotoxicity. Specifically, the NCI-H460 cancer cell line was seeded at 3×10³ cells per well. Then, the cells were infected with OTS-418 at an MOI of 0.02 (0.02 pfu/cells). After 2 hours, co-treatment with GCV at a concentration of 6 µM or 60 µM was performed. Culture was performed for 72 hours, and then cytotoxicity was analyzed using a CCK8 kit (Cell Counting Kit 8).

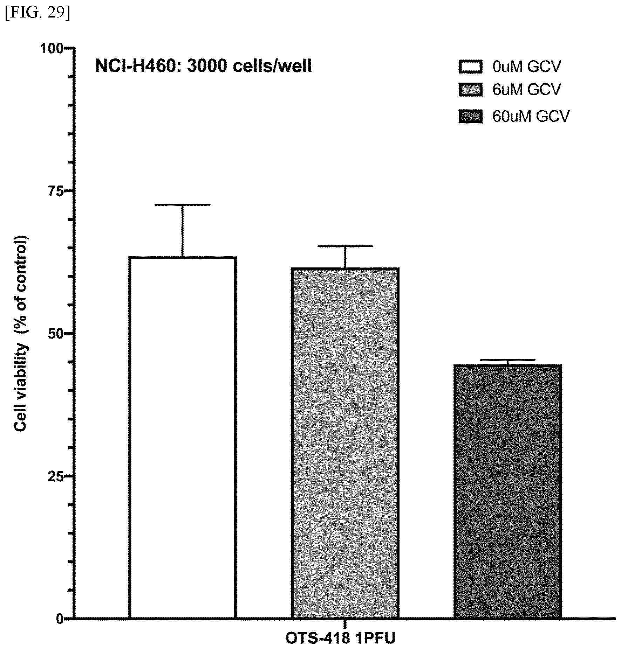

As a result, it was identified that in a case where co-treatment of OTS-418 and GCV was performed, cytotoxicity of OTS-418 increased due to GCV In particular, in a case where co-treatment of OTS-418 and GCV at a concentration of 60 µM was performed, the NCI-H460 cancer cell line was additionally killed by about 20% or higher (FIG. 29). From these results, it was identified that cytotoxicity increased in a case where co-administration of OTS-418 and GCV was performed.

### Example 11. Identification of anticancer effect of recombinant vaccinia virus (WOTS-418) and hydroxyurea in human breast cancer cell line-transplanted mice: MDA-MB-231

Balb/c nu/nu mice (female, 8-week-old) purchased from ORIENT BIO (Busan, Korea) were subjected to a one-week acclimatization period, and then xenografted with a human breast cancer cell line (MDA-MB-231) at 5×10⁶ cells. The tumor volume was observed until it reached 50 mm³, and then drug administration was started. After 31 days, the tumor volume was measured.

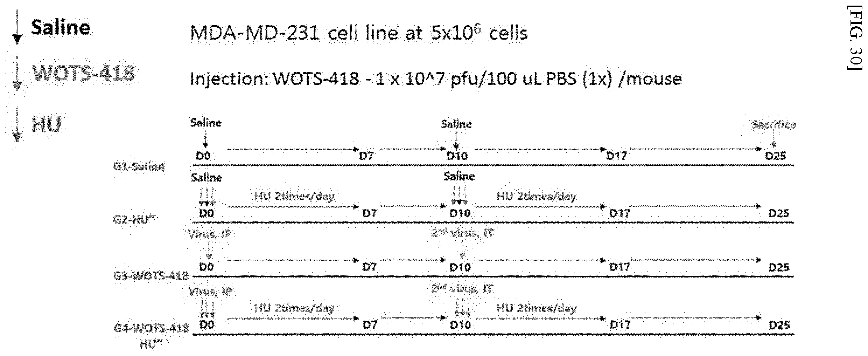

The produced human breast cancer cell line-transplanted mice were divided into 4 groups (n=5). The group receiving intraperitoneal administration of saline was set as a control group, and the group receiving hydroxyurea (HU, 30 mg/kg), the group receiving WOTS-418 (1×10⁷ pfu), and the group receiving both WOTS-418 and HU were classified as experimental groups. WOTS-418 was administered a total of 2 times (D0, D10), in which the administration was performed intraperitoneally on day 0, and then intratumorally on day 10. Hydroxyurea was administered intraperitoneally twice a day on a daily basis (6 times/week) (FIG. 30).

The tumor volume was measured for 31 days. As a result, for the control group, the tumor volume increased slowly, and then dramatically increased starting after day 17 so that the tumor volume on day 31 was larger than the initial tumor volume by 6-fold or higher. In addition, the tumor volume in the mice of the group having received HU gradually increased so that the tumor volume on day 31 was larger than the initial tumor volume by 5-fold or higher.

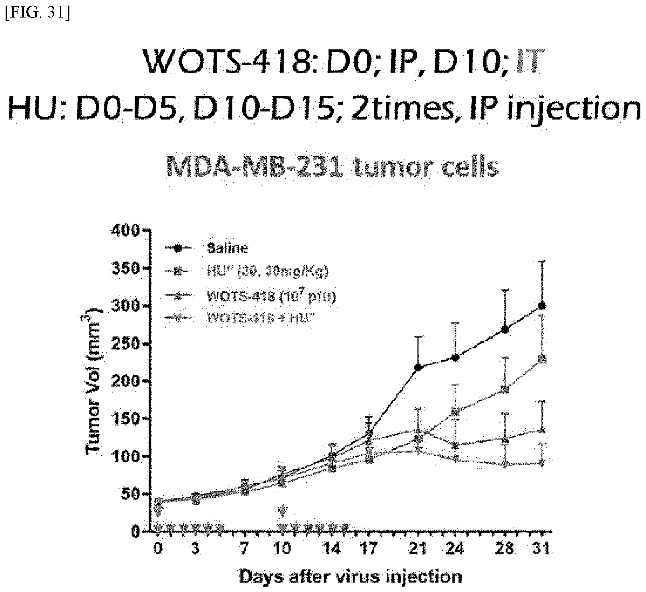

On the other hand, for the tumor volume in the mice of the experimental group having received WOTS-418, it was observed that tumor growth was inhibited starting from day 14 and the tumor remained unchanged until day 31. In addition, for the tumor volume in the mice of the experimental group having received WOTS-418 and hydroxyurea, it was observed that tumor growth was inhibited starting from day 17 and a decreasing tendency in tumor volume was shown (FIG. 31).

From these results, it was found that WOTS-418 was effective in inhibiting tumor growth, and it was identified that although tumor growth was not inhibited in a case where treatment with hydroxyurea alone was performed, an anticancer effect was improved in a case where co-administration of WOTS-418 and hydroxyurea was performed, as compared with a case where administration of WOTS-418 or hydroxyurea alone was performed.

### Example 12. Identification of anticancer effect of recombinant vaccinia virus (WOTS-418) and hydroxyurea in mouse renal cancer cell line-transplanted mice: Renca

Balb/c nu/nu mice (female, 8-week-old) purchased from ORIENT BIO (Busan, Korea) were subjected to a one-week acclimatization period, and then allografted with a mouse renal cancer cell line (Renca, Korea Cell Line Bank). The tumor volume was observed until it reached 100 mm³, and then drug administration was started. After 28 days, the tumor volume was measured.

The produced mouse renal cancer cell line-transplanted mice were divided into 3 groups (n=7). The group receiving intraperitoneal administration of saline was set as a control group, and the group receiving single doses of WOTS-418 and hydroxyurea (1×10⁶ pfu, 30 mg/kg), and the group receiving multiple doses of WOTS-418 and hydroxyurea (1×10⁶/1×10⁷ pfu, 30 mg/kg) were classified as experimental groups. WOTS-418 was administered intraperitoneally on days 0 and 14, and hydroxyurea was administered intraperitoneally twice a day on a daily basis (6 times/week).

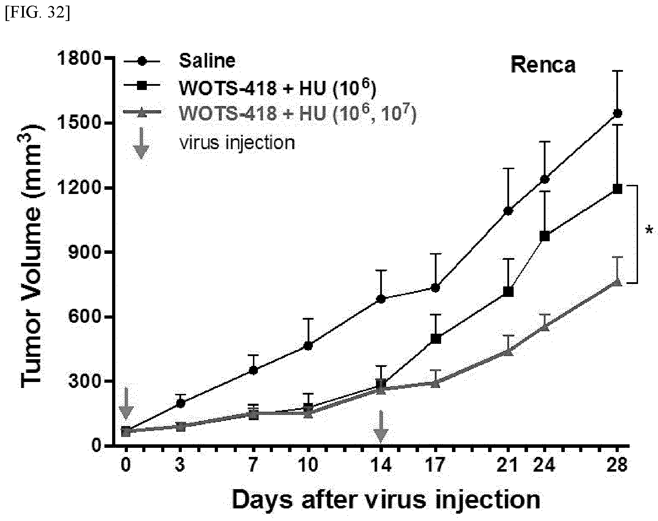

As a result, it was identified that the tumor volume in the mice of the experimental group having received WOTS-418 and hydroxyurea was remarkably suppressed as compared with the control group, and it was identified that the tumor volume was further suppressed in the group having received multiple doses as compared with the group having received single doses. In addition, it was identified that an anticancer effect was enhanced in the group having received multiple doses as the concentration of WOTS-418 increased upon its second administration (FIG. 32).

### Example 13. Identification of anticancer effect of recombinant vaccinia virus (WOTS-418) and hydroxyurea in mouse colorectal cancer cell line-transplanted mice: CT-26

### Experimental Example 13.1. Production of mouse colorectal cancer cell-transplanted mice and drug administration

Balb/c mice (female, 8-week-old) purchased from ORIENT BIO (Busan, Korea) were subjected to a one-week acclimatization period, and then allografted with a mouse colorectal cancer cell line (CT-26, Korea Cell Line Bank). The tumor volume was observed until it reached 100 mm³, and then drug administration was started.

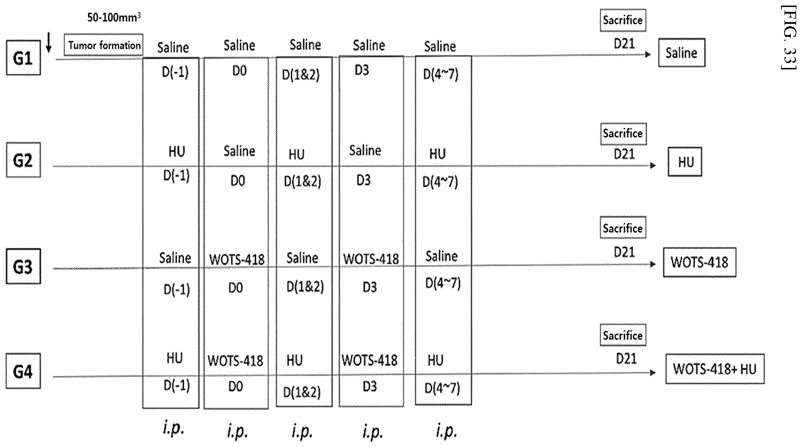

The produced mouse colorectal cancer cell-transplanted mice were divided into 4 groups (G1, G2, G3: n=15, and G4: n=14). The group receiving intraperitoneal administration of saline was set as a control group, and the group receiving intraperitoneal administration of hydroxyurea (30 mg/kg) alone, the group receiving two intraperitoneal administrations (1×10⁸ pfu, i.p., D0, D3) and two intratumoral administrations (1×10⁷ pfu, i.t., D14, D21) of WOTS-418 alone, and the group receiving co-administration of WOTS-418 and hydroxyurea at the same doses and regimens were classified as experimental groups. WOTS-418 was administered intraperitoneally on days 0 and 3, followed by intratumoral administration on days 14 and 21, and hydroxyurea was administered intraperitoneally once a day on a daily basis (6 times/week) starting from immediately before virus administration to day 26 (FIG. 33).

On day 25, the tumor volume was measured and the mice were sacrificed. Then, immune cells were isolated therefrom and subjected to interferon-gamma assay.

### Experimental Example 13.2. Identification of changes in tumor volume

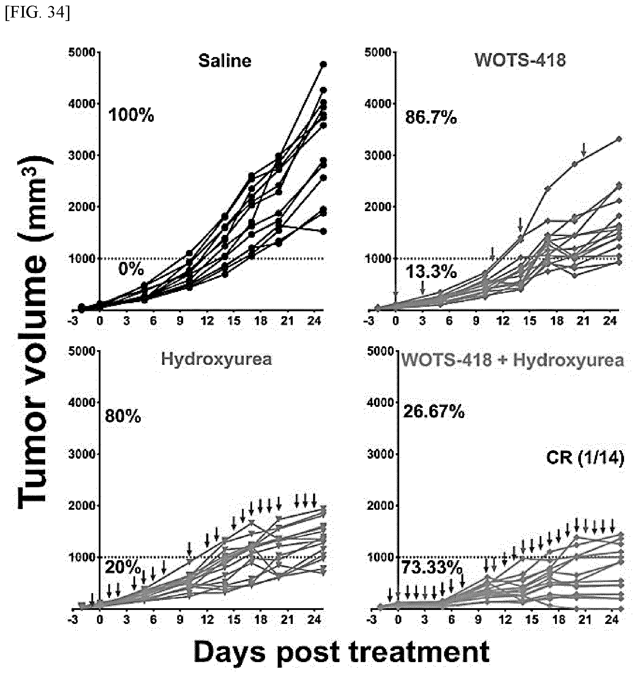

Drug administration was performed on the mice of each group in Experimental Example 13.1, and then the tumor volume was measured on day 25. As a result, it was identified that as compared with the control group, the mice of the experimental group having received administration of WOTS-418 alone, the experimental group having received hydroxyurea alone, and the experimental group having received co-administration of WOTS-418 and hydroxyurea showed suppressed tumor volume. In particular, it was identified that the tumor volume was remarkably suppressed in the mice of the experimental group having received co-administration of WOTS-418 and hydroxyurea (FIG. 34).

### Experimental Example 13.3. Interferon-gamma assay

Drug administration was performed on the mice of each group in Experimental Example 13.1. Then, immune cells were isolated therefrom and subjected to interferon-gamma assay.

Specifically, CD8+ T cells in the spleen of the mice of each group were isolated using (Ly-2) MicroBeads kit and magnetic-activated cell sorting (MACS), and then co-cultured with CT-26 cell line (at 1×10⁴ cells). CD8+ T cells secreting IFN-γ were subjected to ELISPOT (MABTECH, Sweden) experiments, and the spots were scanned and counted by LK Bioscience (Seoul, Korea).

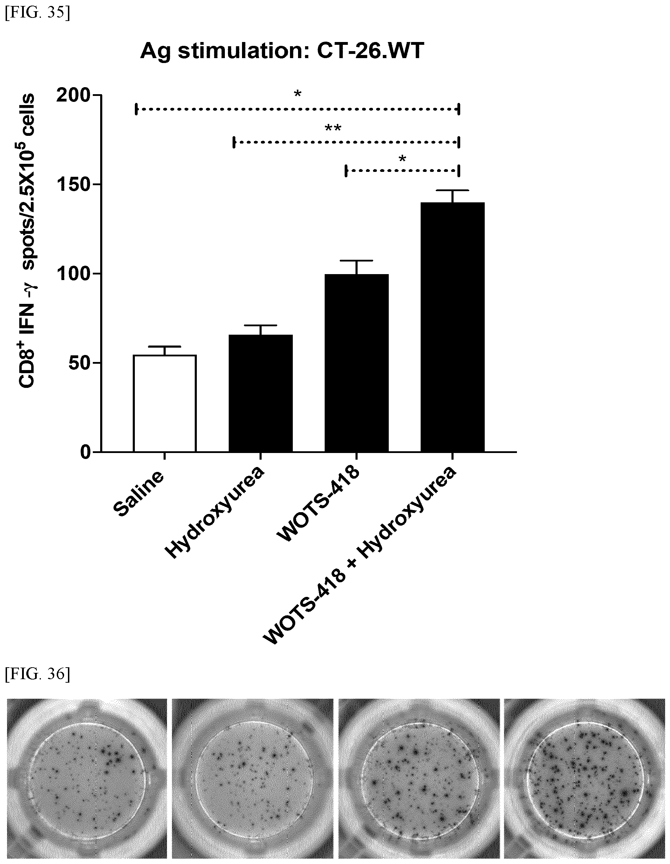

As a result, it was identified that as compared with CD8+ T cells isolated from the spleen of the control mice, CD8+ T cells secreting IFN-γ were abundant in CD8+ T cells isolated from the spleen of the mice of the experimental group having received administration of WOTS-418 alone, the experimental group having received hydroxyurea alone, and the experimental group having received co-administration of WOTS-418 and hydroxyurea. In particular, it was identified that the tumor volume was remarkably suppressed in the mice of the experimental group having received co-administration of WOTS-418 and hydroxyurea (FIGS. 35 and 36).

## Claims

1. An oncolytic virus, comprising:
a nucleotide sequence that encodes a polypeptide including an effector domain,
wherein the effector domain is represented by SEQ ID NO: 1 and derived from herpes simplex virus thymidine kinase (HSV-TK).

2. The oncolytic virus of claim 1, wherein the HSV is herpes simplex virus type 1 (HSV1).

3. The oncolytic virus of claim 1, wherein the polypeptide further has a sequence of 0 to 231 amino acids which is linked to the C-terminus of the effector domain.

4. The oncolytic virus of claim 1, wherein the polypeptide further has a sequence of 36, 82, or 231 amino acids which is linked to the C-terminus of the effector domain.

5. The oncolytic virus of claim 1, wherein the polypeptide consists of an amino acid sequence represented by SEQ ID NO: 1, 3, 5, 7, 9, 11, or 13.

6. The oncolytic virus of claim 1, wherein the nucleotide sequence encoding the polypeptide is a nucleotide sequence represented by SEQ ID NO: 2, 4, 6, 8, 10, 12, or 14.

7. The oncolytic virus of claim 1, wherein the oncolytic virus is derived from adenovirus, herpes simplex virus, lentivirus, retrovirus, adeno-associated virus, vaccinia virus, or poxvirus.

8. The oncolytic virus of claim 1, wherein the oncolytic virus is derived from a vaccinia virus.

9. A pharmaceutical composition for preventing or treating cancer, comprising as an active ingredient:
the oncolytic virus of any one of claims 1 to 8.

10. The pharmaceutical composition of claim 9, wherein the cancer is any one selected from the group consisting of lung cancer, colorectal cancer, prostate cancer, thyroid cancer, breast cancer, brain cancer, head and neck cancer, esophageal cancer, skin cancer, thymic cancer, gastric cancer, colon cancer, liver cancer, ovarian cancer, uterine cancer, bladder cancer, rectal cancer, gallbladder cancer, biliary tract cancer, pancreatic cancer, non-small cell lung cancer, bone cancer, intraocular melanoma, perianal cancer, fallopian tube carcinoma, endometrial carcinoma, cervical cancer, vaginal carcinoma, vulvar carcinoma, Hodgkin's disease, small intestine cancer, endocrine adenocarcinoma, parathyroid cancer, adrenal cancer, soft tissue sarcoma, urethral cancer, penile cancer, chronic leukemia, acute leukemia, lymphocytic lymphoma, kidney cancer, ureteral cancer, renal cell carcinoma, renal pelvis carcinoma, central nervous system tumor, primary central nervous system lymphoma, spinal cord tumor, brainstem glioma, pituitary adenoma, and a combination thereof.

11. A pharmaceutical composition for preventing or treating cancer, comprising as active ingredients:
the oncolytic virus of any one of claims 1 to 8; and
ganciclovir (GCV) or aciclovir (ACV).

12. The pharmaceutical composition of claim 11, wherein the oncolytic virus and the GCV or ACV, which are included in the pharmaceutical composition, are administered simultaneously or sequentially.

13. The pharmaceutical composition of claim 11, wherein the GCV or ACV is administered at a dose of 0.1 µg/kg/day to 50 mg/kg/day.

14. The pharmaceutical composition of claim 11, wherein the cancer is any one selected from the group consisting of lung cancer, colorectal cancer, prostate cancer, thyroid cancer, breast cancer, brain cancer, head and neck cancer, esophageal cancer, skin cancer, thymic cancer, gastric cancer, colon cancer, liver cancer, ovarian cancer, uterine cancer, bladder cancer, rectal cancer, gallbladder cancer, biliary tract cancer, pancreatic cancer, non-small cell lung cancer, bone cancer, intraocular melanoma, perianal cancer, fallopian tube carcinoma, endometrial carcinoma, cervical cancer, vaginal carcinoma, vulvar carcinoma, Hodgkin's disease, small intestine cancer, endocrine adenocarcinoma, parathyroid cancer, adrenal cancer, soft tissue sarcoma, urethral cancer, penile cancer, chronic leukemia, acute leukemia, lymphocytic lymphoma, kidney cancer, ureteral cancer, renal cell carcinoma, renal pelvis carcinoma, central nervous system tumor, primary central nervous system lymphoma, spinal cord tumor, brainstem glioma, pituitary adenoma, and a combination thereof.

15. A method for producing an oncolytic vaccinia virus that includes a gene encoding mutated HSV-TK, the method comprising steps of:
i) removing TK gene from a vaccinia virus and allowing the vaccinia virus to recombine with wild-type HSV-TK gene; and
ii) performing continuous subculture of the recombinant vaccinia virus in the presence of bromodeoxyuridine (BrdU) in a host cell.

16. The method of claim 15, wherein the wild-type HSV-TK gene is a nucleotide sequence represented by SEQ ID NO: 16.

17. The method of claim 15, wherein the oncolytic vaccinia virus has no TK activity and phosphorylates GCV or ACV.

18. A method for treating cancer, comprising:
a step of administering the oncolytic virus of any one of claims 1 to 8.

19. A use of the oncolytic virus of any one of claims 1 to 8 for the treatment of cancer.

20. A use of the oncolytic virus of any one of claims 1 to 8 for the manufacture of a medicament for treating cancer.

21. A method for treating cancer, comprising:
a step of administering the pharmaceutical composition of any one of claims 11 to 14.

22. A use of the pharmaceutical composition of any one of claims 11 to 14 for the treatment of cancer.

23. A use of the pharmaceutical composition of any one of claims 11 to 14 for the manufacture of a medicament for treating cancer.
